# EUROPEAN PATENT APPLICATION

(11) **EP 0 872 486 A1**
(43) Date of publication of application: **21.10.1998**
(21) Application number: 98201097.7
(22) Date of filing: 07.04.1998
(51) Int. Cl.: C07K 14/455, C07K 16/20, A61K 39/012, C12N 15/30, G01N 33/569, C12Q 1/68

(54) **Eimeria proteins as vaccines**

(30) Priority: 09.04.1997 EP 97302447
(71) Applicant: Akzo Nobel N.V., 6824 BM Arnhem (NL)
(72) Inventor: Vermeulen, Arno N., 5431 HH Cuijk (NL); Clercx-Breed, Dominique G.J., 6512 AD Nijmegen (NL)
(74) Representative: Mestrom, Joannes Jozef Louis

(57) **Abstract**

Compositions comprising *Eimeria* proteins or variants/fragments of such proteins can be used to produce a coccidiosis vaccine.

The proteins are present in the hydrophilic phase of a Triton X-1 14 extract of *Eimeria* sporozoites and have molecular masses of 26-30 kDa ± 5 kDA when determined by SDS PAGE under reducing conditions.

## Description

The present invention relates, *inter alia,* to coccidiosis vaccines.

Parasitic protozoa belonging to the genus *Eimeria* are the causative agents of intestinal coccidiosis, an enteritis which affects birds. This causes significant economic loss, especially to the poultry industry. (For the purposes of the present application, the term "poultry" is taken to mean birds that serve as sources of eggs or meat. It includes, *inter alia,* chickens, turkeys, ducks, geese, guinea fowl, pheasants, pigeons and pea fowl). Nowadays, coccidiosis is mainly controlled by the use of antibiotic drugs in the feed. The rapid emergence of drug resistant strains (Chapman 1993) and the prohibitive costs of development and registration of a novel drug have led to increased interest in the development of an alternative method of control. The development of effective vaccines has therefore been desirable for many years. However only partial success has been obtained.

Currently available vaccination strategies consist of controlled infections with either virulent or live attenuated parasites (Shirley 1993). For reasons of safety and cost, the most desirable method of immunoprophylaxis against coccidiosis appears to be the use of a subunit vaccine. Although many attempts have been made to immunize chickens against coccidiosis with fractions of parasite material (Murray *et al.* 1986, McKenzie & Long 1986) or recombinant *Eimeria* proteins (Danforth *et al.* 1989, Jenkins *et al.* 1991) only limited protection against challenge infection could be achieved. The parasite stages responsible for the induction of protective immunity are generally thought to be early asexual developmental stages (Jenkins *et al.* 1991). Initially, selection of candidate antigens was performed using antibodies from immune chickens but, in view of the fundamental role of cell mediated responses in protective immunity (reviewed in Lillehoj & Trout 1993, Rose 1996), attention has now focused on screening antigens for their ability to stimulate specific T-cell responses (Dunn *et al.* 1995).

It has been reported previously that around 8 days after a primary *E. tenella* infection in chickens parasite-specific T-cells circulate in the peripheral blood (Breed *et al.* 1996). These cells appeared to be primed to proliferate and/or to produce IFN-γ in response to triggering with total sporozoite antigens (Breed *et al.* 1997).

According to the present invention there is provided a composition free from intact *Eimeria* parasites, which comprises one or more proteins or fragments or variants thereof; wherein said proteins:
(a) are present in the hydrophilic phase of a Triton X-114 extract of *Eimeria* sporozoites,
(b) have molecular masses of 26-30 kDa ± 5 kDa (i.e. 21-35 kDa) when determined by SDS-PAGE under reducing conditions.

The present inventors have found that such a composition can be used to provide a vaccine which provides a significant degree of protection to birds (preferably poultry) against *Eimeria* mediated disorders. For example, protection against the formation of cecal lesions can be achieved in birds immunised with such a vaccine, when subjected to subsequent challenge with *Eimeria* parasites.

This finding could not have been predicted from previous studies. Although various groups have disclosed *Eimeria* derived proteins which might, by chance, have molecular masses within the 26-30 kDa ± 5kDa range disclosed above, these proteins are quite different from the proteins of the present invention.

For example, in EP-A- 0231537 (Newman *et al*) a 25 kDa surface protein is disclosed. However under reducing conditions this splits to form two bands on SDS-PAGE of about 17 and about 8 kDa, whereas the proteins of the present invention had relative molecular masses of at least 21kDa when run under reducing conditions.

Bouvier *et al* (*J. Biol. Chem.* (1985) 260(29); pp15504-15509) teach that using Triton X114 extraction amphiphilic proteins (membrane-associated) are only detected in the detergent phase and not in the hydrophilic phase.

In US-A-4710377 (Schenkel *et al*) antigens are disclosed with molecular masses of about 28 and 26 kDa. However these are amphiphilic outer-membrane components and would not therefore be present in the hydrophilic phase of a Triton X-114 extract which could be used to prepare proteins of the present invention.

*Eimeria* proteins that are amphiphilic are also disclosed in WO92/04461 (Jacobson *et al*)*,* EP-A-0324648 (Liberator *et al*), AU-A-28542/49 (Turner *et al*)*,* EP-A-0344808 (Alternburger *et al*) and EP-A-0167443 (Murray *et al*).

It is also important to bear in mind that there does not appear to be a direct correlation between the ability of proteins to stimulate T-cell responses following a primary *Eimeria* infection and the ability of proteins to provide a protective response to poultry in vaccination studies.

A composition of the present invention will normally be free of one or more *Eimeria* proteins which occur in *Eimeria* in nature. Preferably it will be substantially free of *Eimeria* proteins, other than *Eimeria* proteins which are present in the hydrophilic phase of a Triton X-114 extract of *Eimeria* sporozoites. Proteins which are membrane-bound in *Eimeria* may therefore be absent.

A substantial part of the proteinaceous material present in the composition (e.g. at least 50% w/w, at least 70% w/w or at least 90% w/w of the total proteins present) may consist of the one or more proteins of the present invention or of fragments or variants thereof.

For example, a plurality of said proteins (or variants or fragments thereof), may be present in such a composition. Two or three such proteins (or variants or fragments thereof) may be present.

Alternatively, only one of these proteins (or a variant or fragment thereof) may be provided. It may be provided in substantially pure form i.e. in a form which is essentially free from any other *Eimeria* proteins. Purification can be achieved by techniques known to those skilled in the art. Such techniques are described in general terms later on.

A suitable purification procedure to provide proteins of the present invention at a sufficient level of purity for sequencing is as follows:

Proteins of the composition can be run on a 12-18% acrylamide (preferably 15%) separating gel in a suitable buffer system in the presence of SDS. After staining of the proteins (e.g. 0.1% Coomassie Brilliant Blue in 45% methanol, 10% acetic acid) bands containing the individual proteins can be excised and digested *in situ* using trypsin.

After extraction of the tryptic peptides and subsequent HPLC purification, the peptides can be sequenced using an automated gas-phase amino-acid sequencer. Using this method internal amino acid sequence can be obtained from as little as 20-50 pmoles of protein. which can be provided from sporozoites using the methods described.

Variants of proteins of the present invention can be provided.

The term "proteins" is used herein to indicate any molecules comprising a plurality of amino acids linked together by peptide bonds. It therefore includes peptides and polypeptides within its scope. It also includes within its scope polypeptide chains which may become separated from larger proteins under reducing conditions due to the cleavage of disulphide bonds.

The term "variants" is used to encompass molecules that have one or more amino acid deletions, insertions and/or substitutions relative to a protein of the present invention, but that retain one or more immunogenic determinants of an *Eimeria* antigen. Variants will have one or more epitopes - preferably complete antigens - that are capable of eliciting an immune response in a host animal (if necessary when combined with a larger molecule).

It will be understood that, for the proteins of the present invention, natural variations in the amino acid sequence can exist between individual *Eimeria* parasites or strains. Such natural variants are within the scope of the present invention, as are variants which do not occur in nature.

Amino acid substitutions which do not substantially alter biological and immunological activities have been described, e.g. by Neurath *et al* in "The Proteins" Academic Press New York (1979). Amino acid replacements between related amino acids or replacements which have occurred frequently in evolution are, *inter alia,* Ser/Ala, Ser/Gly, Asp/Gly, Asp/Asn. Ile/Val (see Dayhof, M.C., Atlas of protein sequence and structure, *Nat. Biomed*, *Res. Found.,* Washington D.C., 1978, vol.5, suppl. 3). Other amino acid substitutions include Asp/Glu, Thr/Ser, Ala/Gly, Ala/Thr, Ser/Asn/Ala/Val, Thr/Phe, Ala/Pro, Lys/Arg, Leu/Ile, Leu/Val and Ala/Glu. Based on this information, Lipman and Pearson have developed a method for rapid and sensitive protein comparison (*Science,* 227, 1435-1441, 1985) and determining the functional similarity between homologous proteins. Amino acid substitutions are within the scope of the invention.

As indicated above, variants are not limited to substitutions but can also/alternatively include deletions and/or insertions which do not remove immunological activity. However deletions or insertions which significantly affect the structural configuration of a protein will generally only be present in variants of the present invention if they do not result in the loss of immunologically signficant *Eimeria* epitopes or antigens.

Preferred variants of the proteins of the present invention have a high degree of sequence identity with said proteins e.g. at least 50%, at least 75%, at least 90% or at least 95% sequence identity.

One way of determining amino acid sequence identity is to align a given amino acid sequence with another amino acid sequence in a manner which achieves the maximum number of matches of amino acids over the length of the given amino acid sequence. The percentage sequence identity will then be (m/t) x 100, where m is the number of matches between the two aligned sequences over the length of the given amino acid sequence and t is the total number of amino acids present in the given amino acid sequence.

Another way of determining sequence identity allows for the introduction of gaps when matching two amino acid sequences (0,1,2,3 or even more gaps may be allowed). This can be done, for example. by using the "Gap" program, which is available from Genetics Computer Group as part of "The Wisconsin Package". This program is based upon an algorithm provided by Smith and Waterman (Advances in Applied Mathematics, 482-489 (1981)).

Both of these ways of determining sequence identity may be used in respect of the present invention, although it is preferred to allow for one or more gaps.

Where high degrees of sequence identity are present there may be relatively few differences in amino acid sequence. Thus for example they may be less than 20, less than 10, or even less than 5 differences.

In any event, it should be appreciated that a large number of variants are within the scope of the present invention. For example, fusion proteins may be provided, whereby a protein of the present invention is linked to another moiety (e.g. in order to increase stability or to aid in purification). Such fusion proteins are described in greater detail later on.

Other variants within the scope of the present invention are any *Eimeria* proteins comprising disulphide bonds from which the proteins of the present invention might be derived by cleavage of said disulphide bonds under reducing conditions.

Fragments of proteins of the present invention are also included within the scope of the present invention. Such fragments are preferably at least 10 amino acids long, more preferably at least 50 or at least 100 amino acids long.

The proteins of the present invention, or fragments or variants thereof, are useful in therapy. They may be provided as a pharmaceutical composition in combination with one or more pharmaceutically acceptable carriers. (For the purposes of the present invention a "pharmaceutically acceptable composition" is a composition suitable for use in veterinary medicine. The term "pharmaceutically acceptable carrier" and similar terms should be construed likewise.)

The pharmaceutical composition may be provided in unit dosage form. It may be provided in a sealed container and will normally be in sterile form.

Preferred pharmaceutical compositions of the present invention are vaccines (especially coccidiosis vaccines). Such vaccines are discussed in greater detail later on.

The present invention is not limited to compositions comprising proteins, fragments or variants thereof of the present invention. Nucleic acid molecules are also provided.

Nucleic acid molecules encoding proteins of the present invention can be provided since amino acid sequences encoding proteins or fragments or variants thereof can be determined by standard methodology. A skilled person can then use the genetic code to identify a large number of nucleic acid molecules which would encode such amino acid sequences.

The "long probe" or "short probe" approach may then be used to isolate and clone specific nucleic acid molecules from *Eimeria.* cDNA molecules may be derived from an appropriate mRNA source. Alternatively, molecules may be synthesized *de novo* using chemical synthesis techniques. Once nucleic molecules have been obtained they may be amplified by standard techniques (e.g. by using PCR).

A nucleic acid molecule according to the present invention can be ligated to one or more heterologous nucleic acid molecules with which it is not associated in nature. resulting in a so-called "recombinant vector". This can be used for the transformation of a suitable host. Useful recombinant vectors are preferably derived from plasmids, bacteriophages, cosmids or viruses.

Specific vectors or cloning vehicles that can be used to clone nucleic acid sequences according to the present invention are known in the art and include, *inter alia,* plasmid vectors such as pBR322, the various pUC, pGEM and Bluescript plasmids; bacteriophages, e.g. lambda gt-ES, Charon 28 and the M13 derived phages, or viral vectors such as SV40, adenovirus or polyoma virus (see also Rodriquez, R.L. and D.T. Denhardt, ed., Vectors: A survey of molecular cloning vectors and their uses, Butterworths, 1988; Lenstra, J.A. *et al.,* Arch, Virol., 110, 1-24, 1990). The methods to be used for the construction of a recombinant vector according to the invention are known to those of ordinary skill in the art and are *inter alia* set forth in Maniatis, T. *et al* (Molecular Cloning A Laboratory Manual, second edition; Cold Spring Harbor Laboratory, 1989).

For example, the insertion of a nucleic acid sequence according to the present invention into a cloning vector can easily be achieved when both the genes and the desired cloning vehicle have been cut with the same restriction enzymes(s) since complementary DNA termini are thereby produced which can anneal and then be ligated together.

Alternatively, it may be necessary to modify the restriction sites that are produced to provide blunt ends either by digesting the single-stranded DNA or by filling in the single-stranded termini with an appropriate DNA polymerase. Subsequently, blunt end ligation with an enzyme such as T4 DNA ligase may be carried out.

If desired, any restriction site may be produced by ligating linkers onto the DNA termini. Such linkers may comprise specific oligonucleotide sequences that encode restriction site sequences. The restriction enzyme cleaved vector and nucleic acid sequence may also be modified by homopolymeric tailing.

"Transformation", as used herein, refers to the introduction of an heterologous nucleic acid sequence into a host cell, irrespective of the method used, for example by direct uptake or by transduction. The heterologous nucleic acid sequence may be maintained through autonomous replication or, alternatively, may be integrated into the host genome. If desired, the recombinant vectors are provided with appropriate control sequences compatible with the designated host. These sequences can regulate the expression of the inserted nucleic acid sequence. In addition to microorganisms, cell cultures derived from multicellular organisms may also be used as hosts.

Vectors of the present invention preferably contain one or more markers that may be used to select for desired transformants, such as ampicillin and tetracycline resistance in pBR322.

A suitable host cell is a microorganism or cell which can be transformed by a nucleic acid sequence encoding a protein of the present invention or a variant or fragment thereof or by a recombinant vector comprising such a nucleic acid sequence. If desired, it can be used to express said protein (or a variant or fragment thereof). The host cell can be of prokaryotic origin, e.g. a bacterium such as an *Escherichia coli*, *Bacillus subtilis,* or *Pseudomonas* species; or of eukaryotic origin, such as yeasts, e.g. *Saccharomyces cerevisiae* or higher eukaryotic cells - e.g. insect, plant or mammalian cells, including HeLa cells and Chinese hamster ovary (CHO) cells. Insect cells include the Sf9 cell line of *Spodopter frugiperda* (Luckow *et al, Biotechnology* 6, 47-55, 1988). Parasites may even be used (e.g. Leishmania. Toxoplasma or *Eimeria*).

Information with respect to cloning and expression in eukaryotic cloning systems can be found in Esser, K., *et al* (Plasmids of Eukaryotes, Springer-Verlag, 1986).

In general, prokaryotes are preferred for the construction of the recombinant vectors useful in the present invention. *E. Coli* K12 strains are particularly useful, especially DH5a or MC1061 strains.

For expression, nucleic acid molecules of the present invention can be introduced into an appropriate expression vector in which said molecules are operably linked to expression control regions. Such control regions may comprise promotors, enhancers, operators, inducers, ribosome binding sites etc. Therefore, the present invention provides a recombinant vector comprising a nucleic acid sequence encoding an *Eimeria* protein of the present invention or a fragment or variant thereof, operably linked to one or more expression control sequences.

It should be understood, of course, that the nucleotide sequences inserted at the selected site of the cloning vector may include nucleotides which are not part of the actual structural gene for a desired expression product, or may include only a fragment of the complete structural gene for a desired expression product as long as the transformed host will produce an expression product having at least one or more antigenic determinants (epitopes) of an *Eimeria* protein antigen.

When the host cells are bacteria, useful expression control sequences which may be used include the Trp promotor and operator (Goeddel, *et al., Nucl. Acids Res.,* 8, 4057, 1980); the *Lac* promotor and operator (Change, *et al, Nature* 275 615 1978); the outer membrane protein promoter (Nakamura, K. and Inouge, M.. *EMBO J.,* 1, 771-775, 1982); the bacteriophage lambda promotors and operators (Remaut, E., *et al., Nucl. Acids Res.,* 11, 4677-4688, 1983); and other expression enhancement and control sequences compatible with the selected host cell.

When the host cells is yeast, illustrative useful expression control sequences include, e.g., mating factors. For insect cells, the polyhedrin or p10 promotors of baculoviruses can be used (Smith, G.E. *et al., Mol. Cell. Biol.* 3, 2156-65, 1983). When the host cell is of mammalian origin illustrative useful expression control sequences include the SV-40 promotor (Berman, P.W. *et al., Science,* 222, 524-527, 1983), the metallothionein promoter (Brinster, R.L., *Nature,* 296, 39-42, 1982) or a heat shock promoter (Voellmy *et al., Proc. Natl. Acad. Sci. USA,* 82, 4949-53, 1985). Alternatively, expression control sequences present in *Eimeria* may also be provided. For maximising gene expression, see also Roberts and Lauer (*Methods in Enzymology,* 68, 473, 1979).

Therefore, the invention further comprises non avian hosts containing a nucleic acid sequence or a recombinant nucleic acid molecule or a recombinant vector described above, capable of producing an *Eimeria* protein by expression of a protein encoded by said nucleic acid sequence.

Immunization of birds against an *Eimeria* infection can be achieved by administering to the birds a protein (or a variant or fragment thereof) according to the present invention as a so-called subunit vaccine. The subunit vaccine according to the invention may comprise a protein (or variant or fragment thereof) in a substantially pure form, optionally in the presence of a pharmaceutically acceptable carrier. The protein (or variant or fragment thereof) can be covalently bound to a heterologous protein (or variant or fragment thereof), which can be of advantage in purification.

Examples of such a heterologous protein are β-galactosidase, protein A, prochymosine, blood clotting factor Xa, etc.

In some cases the ability to raise protective immunity using these proteins (or variants or fragments thereof) *per se* may be low. Small fragments (sometimes referred to as "haptens") may therefore be conjugated to carrier molecules in order to raise their immunogenicity. Suitable carriers for this purpose are macromolecules. These include natural polymers (e.g. proteins such as key hole limpet hemocyanin and albumin, toxins), synthetic polymers like polyamino acids (polylysine, polyalanine), or micelles of amphiphilic compounds like saponins. Alternatively these fragments may be provided as polymers thereof, preferably linear polymers.

If required, the proteins (or variants or fragments thereof) according to the invention which are to be used in a vaccine can be modified *in vitro* or *in vivo,* for example by glycosylation, amidation, carboxylation or phosphorylation.

An alternative to subunit vaccines is live vaccines. A nucleic acid molecule according to the invention can be introduced by recombinant DNA techniques into a microorganism (e.g. a bacterium or virus) in such a way that the recombinant microorganism is still able to replicate, thereby expressing a polypeptide coded by the inserted nucleic acid sequence and eliciting an immune response in the infected host bird. M.A. Barry *et al* in *Nature* (1995), 377;pp632-635 teach how to prepare vaccines using nucleic acid molecules.

A preferred embodiment of the present invention is a recombinant vector virus comprising an heterologous nucleic acid sequence described above, capable of expressing the DNA sequence in (a) host cell(s) or host bird infected with the recombinant vector virus. The term "heterologous" indicates that the nucleic acid sequence according to the invention is not normally present in nature in the vector virus.

Furthermore. the invention also comprises (a) host cell(s) or cell culture infected with the recombinant vector virus, capable of producing the *Eimeria* protein (or variant or fragment thereof) by expression of the nucleic acid sequence.

For example the well known technique of *in vivo* homologous recombination can be used to introduce an heterologous nucleic acid sequence according to the invention into the genome of the vector virus.

Firstly, a DNA fragment corresponding with an insertion region of the vector genome, i.e. a region which can be used for the incorporation of an heterologous sequence within disrupting essential functions of the vector such as those necessary for infection or replication, is inserted into a cloning vector according to standard recombinant DNA techniques. Insertion regions have been reported for a large number of microorganisms (e.g. EP 80,806; EP 110,385; EP 83,286; EP 314,569; WO 88/02022, WO 88/07088, US 4,769,330 and US 4,722,848).

Secondly, if desired, a deletion can be introduced into the insertion region present in the recombinant vector molecule obtained from the first step. This can be achieved for example by appropriate exonuclease III digestion or restriction enzyme treatment of the recombinant vector molecule from the first step.

Thirdly, the heterologous nucleic acid sequence is inserted into the insertion-region present in the recombinant vector of the first step or in place of the DNA deleted from said recombinant vector. The insertion region DNA sequence should be of appropriate length as to allow homologous recombination with the vector genome to occur.

Thereafter, suitable cells can be infected with wild-type vector virus or transformed with vector genomic DNA in the present of the recombinant vector containing the insertion flanked by appropriate vector DNA sequences whereby recombination occurs between the corresponding regions in the recombinant vector and the vector genome. Recombinant vector progeny can now be produced in cell culture and can be selected for example genotypically or phenotypically, e.g. by hybridization, detecting enzyme activity encoded by a gene co-integrated along with the heterologous nucleic acid sequence, or detecting the antigenic heterologous protein, expressed by the recombinant vector immunologically.

Next, this recombinant microorganisms can be administered to birds for immunization. It can then maintain itself for some time, or even replicates in the body of the inoculated animal, expressing *in vivo* a protein coded for by the inserted nucleic acid sequence according to the invention, resulting in the stimulation of the immune system of the inoculated animal. Suitable vectors for the incorporation of a nucleic acid sequence according to the invention can be derived from viruses such as pox viruses, e.g. vaccinia virus (EP 110,385; EP 83,286, US 4,769,330 and US 4,722,848) or fowl pox virus (WO 88/02022), herpes viruses such a HVT (WO 88/07088) or Marek's Disease virus, adenovirus or influenza virus, or bacteria such as *E. coli* or specific *Salmonella* species. With recombinant microorganisms of this type, the protein synthesized in the host animal can be exposed as a surface antigen. In this context fusion of the protein with OMP proteins (or variants or fragments thereof), or pilus proteins (or variants or fragments thereof) of for example *E. coli* or synthetic provision of signal and anchor sequences which are recognized by the organism are conceivable. It is also possible that the *Eimeria* protein, if desired as part of a larger whole, is released inside the animal to be immunized. In all of these cases it is also possible that one or more immunogenic products will find expression which generate protection against various pathogens and/or against various antigens of a given pathogen.

A vector vaccine according to the invention can be prepared by culturing a recombinant bacterium or a host cell infected with a recombinant vector comprising a nucleic acid sequence according to the invention, whereafter recombinant bacteria or vector containing cells and/or recombinant vector viruses grown in the cells can be collected, optionally in a pure form, and formed into a vaccine optionally in a pure form, and formed into a vaccine optionally in a lyophilised form.

Host cells transformed with a recombinant vector according to the invention can also be culture under conditions which are favourable for the expression of a protein coded by said nucleic acid sequence. Vaccines may be prepared using samples of the crude culture, host cell lysates or host cell extracts, although in another embodiment more purified proteins according to the invention are formed into a vaccine, depending on its intended use. In order to purify the proteins produced, host cells transformed with a recombinant vector according to the invention are cultured in an appropriate medium and the proteins produced are isolated from such cells, or from the medium if the protein (or variant or fragment thereof) is excreted. Proteins excreted into the medium can be isolated and purified by standard techniques, e.g. salt fractionation, centrifugation, ultrafiltration, chromatography, gel filtration or immuno affinity chromatography, whereas intracellular proteins can be isolated by first collecting said cells, disrupting the cells, for example by sonication or by other mechanically disruptive means such as French press, followed by separate of the proteins from the other intracellular components and forming the proteins into a vaccine. Cell disruption could also be achieved by chemical means (e.g. by using EDTA or by using detergents, such as Triton X114) or by enzymatic means, such as lysozyme digestion.

Antibodies or derivatives thereof (e.g. fragments such as Fab, F(ab')₂ or Fv fragments), which are directed against a protein according to the invention have potential uses in passive immunotherapy, diagnostic immunoassays and in the generation of anti-idiotypic antibodies. Preferably these are specific for the *Eimeria* proteins of the present invention or variants/fragments thereof. Serum comprising antibodies or derivatives thereof may also be provided.

The *Eimeria* proteins (or variants or fragments thereof) as characterized above can be used to produce antibodies, which may be polyclonal, monospecific or monoclonal (or derivatives thereof). If polyclonal antibodies are desired, techniques for producing and processing polyclonal sera are known in the art (e.g. Mayer and Walter, eds. *Immunochemical Methods in Cell and Molecular Biology,* Academic Press, London, 1987). Monospecific antibodies to an immunogen can be affinity purified from polyspecific antisera by a modification of the method of Hall *et al (Nature,* 311, 379-387, 1984). Monospecific antibody, as used herein, is defined as a single antibody species or multiple antibody species with homogeneous binding characteristics for the relevant antigen. Homogeneous binding, as used herein, refers to the ability of the antibody species to bind to a specific antigen or epitope.

Monoclonal antibodies, reactive against the *Eimeria* proteins (or variants or fragments thereof) according to the present invention, can be prepared by immunizing inbred mice by techniques known in the art (Kohler and Milstein, *Nature,* 256, 495-497, 1975). Hybridoma cells are selected by growth in hypoxanthine, thymidine and aminopterin in an appropriate cell culture medium such as Dulbecco's modified Eagle's medium. Antibody producing hybridomas are cloned, preferably using the soft agar technique of MacPherson, (Soft Agar Techniques, Tissue Culture Methods and Applications, Kruse and Paterson, eds., Academic Press, 276, 1973). Discrete colonies are transferred into individual wells of culture plates for cultivation in an appropriate culture medium. Antibody producing cells are identified by screening with the appropriate immunogen. Immunogen positive hybridoma cells are maintained by techniques known in the art. Specific anti-monoclonal antibodies are produced by cultivating the hybridomas *in vitro* or preparing ascites fluid in mice following hybridoma injection by procedures known in the art.

Anti-idiotypic antibodies are immunoglobulins which carry an "internal image" of the antigen of the pathogen against which protection is desired and can be used as an immunogen in a vaccine (Dreesman *et al., J. Infect. Disease,* 151, 761, 1985). Techniques for raising anti-idiotypic antibodies are known in the art (MacNamara *et al., Science* 226, 1325, 1984).

The vaccine according to the invention can be administered in a conventional active immunization scheme: single or repeated administration in a manner compatible with the dosage formulation, and in such amount as will be prophylactically effective, i.e. the amount of immunizing antigen or recombinant microorganism capable of expressing said antigen that will induce immunity in birds (especially poultry) against challenge by virulent *Eimeria* parasites. Immunity is defined as the induction of a significant level of protection in a population of birds after vaccination compared to an unvaccinated group.

A vaccine comprising the protein of the invention may reduce the number of oocysts shedded by the infected animals. Normally, the shedded oocysts will infect other animals in the flock. A decrease in the number of oocysts shedded will then also give a decrease in the number of animals which is subsequently infected and also a decrease in the number of oocysts shedded will give rise to a lesser infective load.

Furthermore, even without effect on the parasite itself, a vaccine may decrease the incidence of disease. This is especially so when the symptoms of the disease are caused by products released by the parasite. Vaccines directed against such products alleviate the symptoms with attacking the parasite.

In any event it is preferred that a vaccine of the present invention is capable of reducing the number of cecal lesions in a bird when challenged with a subsequent *Eimeria* infection.

For live viral vector vaccines the dose rate per chicken may range from 10³ to 10⁸ pfu (but even <1000 pfu might be sufficient e.g. for HVT). A typical subunit vaccine according to the invention comprises 0.1 to 100µg of the protein (or variant or fragment thereof) according to the invention. Preferably at least 5µg will be present. Such vaccines can be administered intradermally, subcutaneously, intramuscularly, intraperitoneally, intravenously, orally or intranasally.

Additionally the vaccine may also contain an aqueous medium or a water containing suspension, often mixed with other constituents in order to increase the activity and/or the shelf life. These constitutents may be salts, pH buffers, stabilizers (such as skimmed milk or casein hydrolysate), emulsifiers, adjuvants to improve the immune response (e.g. oils, muramyl dipeptide, aluminium hydroxide, saponin, polyanions and amphiphatic substances) and/or preservatives. A preferred adjuvant is Quil A. This may be administered at a level of around 150µg/dose (for example).

A vaccine comprising a protein, variant or fragment of the present invention may also comprise other immunogenic proteins of *E. tenella* or immunogenic proteins of other *Eimeria* species. Such a combination vaccine can decrease the parasitic load in a flock of birds and can increase the level of protection against coccidiosis.

A vaccine according to the present invention may also contain immunogens related to other pathogens of birds. or may contain nucleic acid sequences encoding these immunogens, like antigens of Marek's Disease virus (MDV), Newcastle Disease virus (NDV), Infectious Bronchitis virus (IBV), Chicken Anemia Agent (CAA), Reo virus, Avian Retro virus, Fowl Adeno virus, Turkey Rhinotracheitis virus or E. *Coli. Thus* a multivalent vaccine may be provided.

The invention also relates to an "immunochemical reagent", which reagent comprises a protein (or variant or fragment thereof) according to the invention. The term "immunochemical reagent" signifies that the protein (or a variant or fragment thereof) according to the present invention is bound to a suitable support or is provided with a labelling substance.

The supports that may be used are, for example, the inner wall of a microtest well or a cuvette, a tube or capillary, a membrane, filter, test strip or the surface of a particle such as, for example, a latex particle, an erythrocyte, a dye sol, a metal sol or metal compound as sol particles. (A sol is a stable suspension of microparticles which as such are not soluble in water, such as metals.)

Labelling substances which can be used are, *inter alia,* a radioactive isotope, a fluorescent compound, an enzyme, a dye sol, metal sol or metal compounds as sol particles.

A nucleic acid molecule according to the invention can also be used to provide specific probes for hybridization to allow the detection of *Eimeria* related nucleic acids in a sample. Preferably such hybridization occurs under stringent conditions (e.g. at a temperature of from about 35 to about 65°C using a salt solution that is about 0.9 molar. However, the skilled person will be able to vary such parameters as appropriate in order to take into account variables such as probe length, base composition, type of ions present, etc.)

The present invention also comprises a test kit comprising nucleic acid molecules of the present invention useful in the diagnosis of an *Eimeria* infection. They can be provided as probes or primers. For example primers may be used in PCR.

The invention further comprises a test kit useful in an immunoassay.

This test kit may contain at least one immunochemical reagent according to the invention. The immunochemical reaction which takes place using such a test kit is preferably a sandwich reaction, an agglutination reaction, a competition reaction or an inhibition reaction.

For carrying out a sandwich reaction, the test kit can comprise, for example, a protein, variant or fragment according to the present invention bonded to a solid support, for example the inner wall of a microtest well. Alternatively it may comprise a labelled protein according to the present invention.

The test kit need not however comprise the aforesaid components. It may comprise one or more antibodies or derivatives thereof.

The present invention will now be described by way of example only with reference to the accompanying drawings.

### MATERIALS AND METHODS

### Experimental design

Fractions of E. *tenella* sporozoite protein were tested for their ability to stimulateT-cells from *E. tenella* infected chickens *in vitro,* as measured by lymphocyte proliferation and MAF-activity in their supernatants. Hereto, chickens were orally inoculated with 1000 sporulated *E. tenella* oocysts, 8 days later PBL were isolated for stimulation *in vitro.*
Sporozoite fractions that stimulatedT-cells were used as vaccine preparations and were tested for their T-cell related immunogenicity and for their efficacy. Vaccination efficacy was assessed by the reduction in cecal lesions after a vigorous *E. tenella* challenge.

### Chickens

Outbred unsexed White Leghorn chickens, raised under specific-pathogen-free conditions. were kept in isolators with free access to food and water. Faeces were monitored weekly to assure that the animals were free of unwanted coccidial infections. For infection chickens were used at 5-7 weeks of age. For vaccination 3 week old chickens were used.

### Parasites and purification of sporozoites

The Weybridge strain of *E. tenella* was used (Shirley 1986). The parasites were passaged at regular intervals through coccidia-free chickens. Handling of oocysts, release of sporocysts and sporozoites from sporulated oocysts was performed as described earlier (Long *et al.* 1976) using 0.4% taurocholate (Sigma, St. Louis, MO, USA) instead of bile salts (Toyama & Kitano 1983). The sporozoites were further purified by nylon wool passage (Larsen *et al.* 1984) and stored as pellets at -70°C.

### Sporozoite protein fractionation

### Triton-X114 extraction

A Triton X-114 extraction was performed to isolate the hydrophilic phase of total sporozoite proteins (HPS) (Bordier 1981). Hereto, 2 x 10⁹ purified E. *tenella* sporozoites were suspended (1 x 10⁹/ml) in 10 mM Tris-HCl, 150 mM NaCl pH 7.4 (TBS) supplemented with protease inhibitors; 1 mM phenylmethyl sulfonyl fluoride (PMSF, Serva, Heidelberg, Germany), 5 µg/ml Aprotinine, 1 µg/ml Leupeptin and 1 µg/ml Pepstatin A and sonified three times 20 seconds at position 7, on ice (using a sonifier from Branson, Soest, The Netherlands). Precondensed Triton X-114 (Serva) was added to the sporozoite suspension to a final concentration of 10% (v/v) and mixed well to dissolve the proteins (total volume 10 ml). Non-solubilised material was pelleted by centrifugation (20 min 12000g at 4°C). The supernatant recovered was layered over a sucrose cushion and incubated 15 min 40°C (phase separation) and spun 10 min 400 g at room temperature (RT). Extraction of the hydrophilic fraction was repeated once in 10% (v/v) and subsequently in 20% (v/v) precondensed Triton X-114. The total protein concentration was determined using the bichinchonic acid (BCA) assay (Pierce Chemicals, Rockford, Illinois, USA). This hydrophilic phase was stored at -70°C until further use.

### Prep-cell fractionation

All procedures were performed at 4°C. Prior to fractionation, HPS was concentrated by acetone precipitation. After centrifugation 10 min 15000g at 4°C and air drying, pellets were dissolved in reducing sample buffer (Laemmli 1970) containing 30 mg/ml dithiotreitol (DTT) and boiled 3 min at 100°C. The hydrophilic proteins were fractionated using a 12% (w/v) poly acrylamide (PAA) separating gel (7 cm) and a 4% (w/v) PAA stacking gel in the 37 mm tube of the Bio-Rad Prep cell apparatus (Bio-Rad Labs, Richmond, CA) according to the manufacturer's protocol. The Prepcell was operated at 40mA, 500V max. Fractions (± 3 ml) were collected overnight and stored at -85°C. Samples of the fractions were diluted once in 2 x strength reducing sample buffer and taken for analysis with sodium-dodecyl-sulphate polyacrylamide gel-electrophoresis (SDS-PAGE) using a 12 % (w/v) PAA gel (Laemmli 1970). The gels were silverstained according to Wray *et al.* (1981). Fifty fractions were combined into 9 fractions based on their relative molecular mass and dialysed against 0.01M phosphate buffered saline (PBS) pH 7.3. The total protein concentration in the pooled fractions was determined using the BCA assay.

### Preparation of sporozoite fractions for the lymphocyte proliferation assay

The pooled fractions and the HPS were treated with Biobeads (Calbiochem, La Jolla, CA) to remove residual detergent. Beads were first washed 5 times with PBS (0.04 M, pH 7.3, 280-300 mOsm/kg) and incubated for 30 min with RPMI 1640 medium (Dutch modification) supplemented with 2 mM L-glutamine. 1 mM Na-pyruvate, 200 IU/ml penicillin, 200 µg/ml streptomycin (RPMI-GPPS), and 2% normal chicken serum (GIBCO; Life Technologies Paisley, United Kingdom). The samples were incubated with the beads for 1 h at RT on a rotating platform and subsequently dialysed against PBS, at 4°C. After acetone precipitation protein pellets were washed with 96% ethanol, air dried and dissolved in RPMI-GPPS.

### Lymphocyte proliferation assay

The proliferative responses of PBL were measured by [³H]thymidine incorporation as described (Breed *et al.* 1996). Briefly, PBL from infected, vaccinated or control chickens (1 x 10⁶ /well) were cultured in round-bottomed 96-well microtiter plates (Nunc, Kampstrump, Denmark) in the presence of either total *E. tenella* sporozoite antigen (equivalent to 3 x 10⁵ sporozoites per well), HPS (10-15 µg/well) or pooled fractions of these (50 µl/well) or with medium alone in a total volume of 150 µl RPMI-GPPS supplemented with 1% (v/v) normal chicken serum (GIBCO; Life Technologies, Paisley, United Kingdom). After 64 h of incubation plates were centrifuged, 80 µl culture fluid was removed and assayed for the presence of MAF-activity (see nitrite assay). Fresh RPMI-GPPS medium was added and cells were pulsed for the final 8 h with 18.5 kBq of [³H]thymidine per well (specific activity 185 GBq/mmol). [³H]thymidine incorporation was measured using a Betaplate 1205 (Wallac, Turku, Finland). Proliferative responses were expressed as stimulation indices ( SI), which represents the ratio of the mean proliferation in counts per minute (CPM) after stimulation to the value of medium controls.

### Nitrite assay

Production of nitric oxide by HD11 chicken macrophages (Beug *et al.* 1979) was used as a measure of MAF activity, most likely due to IFN-γ (Breed *et al.* 1997). Nitric oxide was quantitated by accumulation of nitrite in the culture medium as described by Breed *et al.* (1997). Briefly, 100 µl HD11 cells (1.5 x 10⁶ /ml RPMI-GPPS + 20% (v/v) fetal calf serum and 2% (v/v) L-arginine solution) were cultured in round-bottomed 96-well plates (Nunc) in the presence of 100 µl lymphocyte supernatants. After 24h incubation the amount of NO₂⁻ in the culture medium was determined by the Griess reaction. Supernatant from Con A activated chicken spleen cells was included for reference. Data are expressed as µM NO₂⁻/1.5 x 10⁶cells/24 h (MAF-activity). The nitrite index (NI) represents the ratio of the mean amount of nitrite measured using supernatants of stimulated cultures to the value of non-stimulated cultures (medium controls).

### Vaccination experiments

The selected antigen preparations were thawed and the volumes were adjusted to obtain 5-10 µg protein/dose (0.5 ml) unless otherwise indicated. To each dose 150 µg/dose Quil A (Superfos Biosector, Vedbaek, Denmark) was added as adjuvant. The different vaccine preparations were injected subcutaneously in groups of ±10 chickens. The control group was injected with adjuvant in PBS. After ±3 weeks chickens were boosted with the same preparation, which was prepared freshly from the frozen antigen stock. Eleven days after vaccination heparinized blood samples (5 ml) were taken by cardiac puncture and assayed for proliferation and MAF-production in response to total E. *tenella* sporozoite antigen (equivalent to 3 x 10⁵ sporozoites per well) or medium alone. Three to four days later all chickens were orally inoculated with 4500 sporulated oocysts of *E. tenella.* One week after this challenge chickens were assayed for the presence of cecal lesions. The severity of the cecal lesions was scored on a scale of 0-4 as described (Johnson & Reid 1970).

### Statistical analysis

Statistical analysis of the lymphocyte proliferation data was performed using Student's *t*-test on Log transformed stimulation indices (Bennett & Riley 1992). For the nitrite assay similar transformation was performed. Differences on group means were considered significant at P values <0.05. Differences between group mean values of lesion scores were analysed using the Student's *t*-test.

### RESULTS

### Pooled hydrophilic fractions of E. tenella sporozoites

The hydrophilic protein phase of purified *E. tenella* sporozoite homogenates (HPS) obtained by TX-114 extraction was fractionated using preparative gel electrophoresis. The relative molecular mass of the proteins in each fraction was estimated using SDS-PAGE analysis. Nine fractions were obtained containing proteins of different relative molecular mass (Table 1). These fractions were tested for their ability to stimulate PBL from *E. tenella* infected chickens.

**Table 1**

| Relative molecular mass of hydrophilic fractions of *E. Tenella* sporozoites. | |
|---|---|
| Fraction | Relative Molecular Mass |
| 1 | ≤ 15,000 |
| 2 | about 16,000-25,000 |
| 3 | about 26,000-30,000 |
| 4 | about 31,000-33,000 |
| 5 | about 34,000-39,000 |
| 6 | about 40,000 |
| 7 | about 40,000-48,000 |
| 8 | about 49,000 |
| 9 | > 50,000 |

### Selection of T-cell stimulating antigen fractions

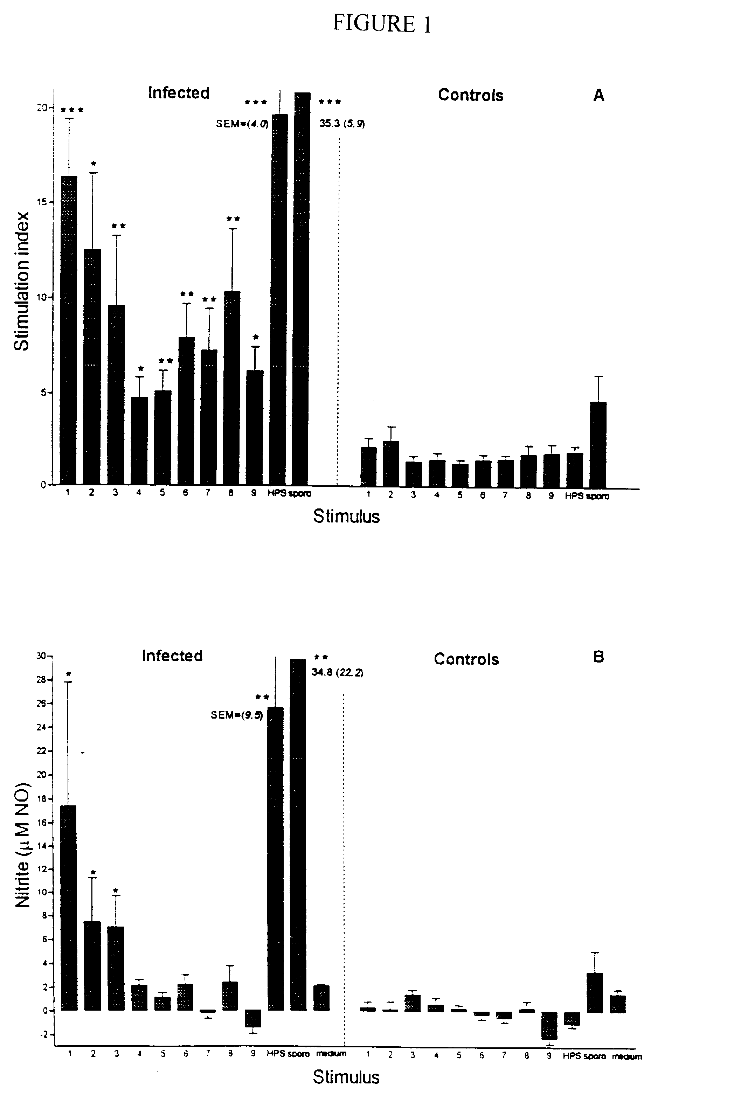

The capacity of the fractions to stimulate T-cell responses in PBL from infected chickens (day 8 p.i.) was measured by lymphocyte proliferation and MAF-activity in their supernatants. The mean stimulation indices are shown in figure 1A. Both antigen-control preparations (total sporozoite antigen and HPS) induced high proliferative responses in the infected animals. All 9 fractions induced significantly higher proliferative responses in PBL from infected animals than in PBL from the uninfected controls. The highest responses were found against fractions 1, 2, 3, and 8. Culture fluids of stimulated PBL were harvested and tested for the presence of MAF-activity using the nitrite assay. MAF-activity, expressed as the amount of nitrite produced by macrophages, are shown in figure 1B. High levels of MAF-activity were measured in culture fluid of PBL stimulated with the antigen-control preparations. The background levels in non-stimulated cultures were low. Only fractions 1, 2 and 3 induced a significantly higher level of MAF-activity in culture fluid of PBL from infected chickens than in those of uninfected chickens. The fractions that stimulated both types of responses (1, 2 and 3) and fraction 8, containing relatively high proliferation inducing capacity and low MAF inducing activity, were selected to determine their vaccine potential.

### Characterization of selected antigen fractions

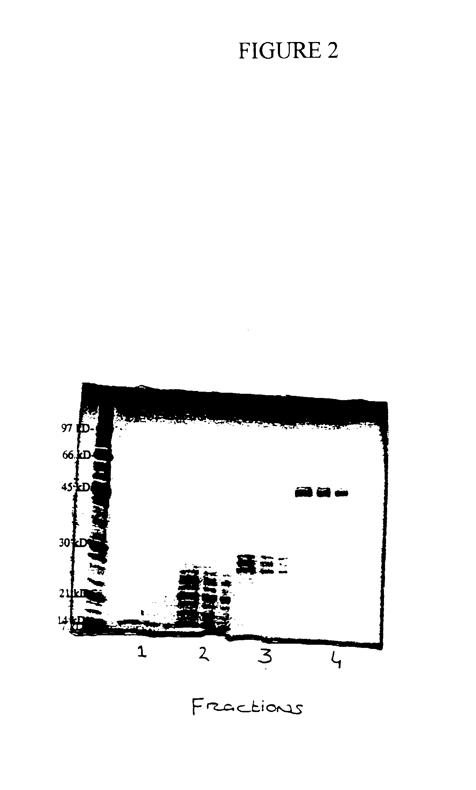

The four selected antigen fractions were analysed using SDS-PAGE. Figure 2 shows the different polypeptides present in the selected fractions. Fraction 1 contained polypeptides with a molecular mass < 15 kD. Fraction 2 showed about nine different polypeptides, two predominant bands of about 16 kD and 22 kD and minor bands ranging from 17-26 kD. Fraction 3 contained 3 polypeptides ranging from 26-30 kD (± 5 kDa, to allow for possible limitations in the measurement techniques used). Fraction 8 showed a predominant polypeptide of about 49 kD.

### Determination of vaccine potential of selected antigen fractions

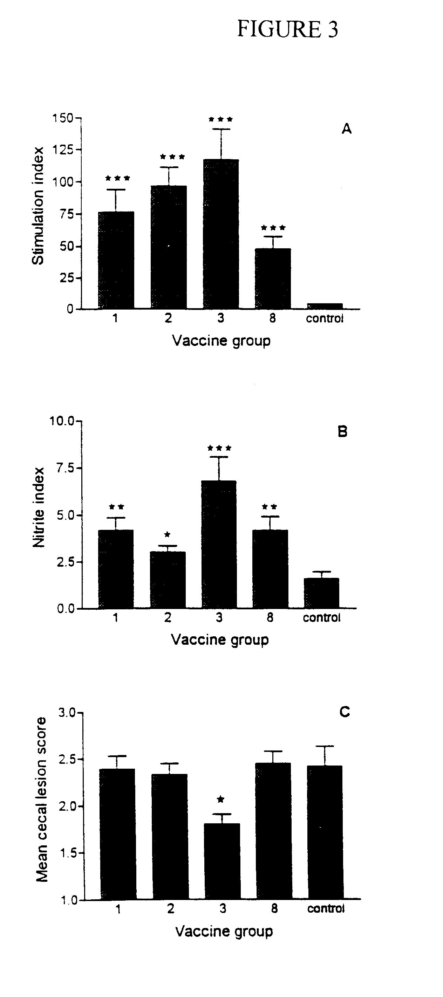

Groups of chickens were immunized with the four selected antigen fractions. Animals received a priming vaccination at day 0 and a booster vaccination at day 21. Eleven days after the booster vaccination (three days before challenge infection) PBL were harvested and stimulated with total sporozoite antigen to determine T-lymphocyte reactivity. Lymphocytes from all four experimental groups exhibited high reactivity upon stimulation with sporozoite antigen with regard to both, stimulation of lymphocyte proliferation and induction of MAF-activity in their supernatants (Fig. 3A and B).
Lymphocytes from chickens vaccinated with fraction 3 showed the highest responses. Fourteen days after booster vaccination all animals were challenged with *E. tenella* sporulated oocysts. Seven days later animals were sacrificed to determine the lesion score in the ceca. One value (present in group 3) was excluded from further analysis, since it was determined that this was the only value outside the 95% confidentiality limit of the total group (n=46; value > mean + 1.96 x SD). Results show that only the group of animals vaccinated with antigen fraction 3 had reduced cecal lesion scores compared to non-vaccinated controls (Fig. 3C). This reduction was statistically significant (P < 0.05).

### Dose-effect response of protective antigen fraction 3

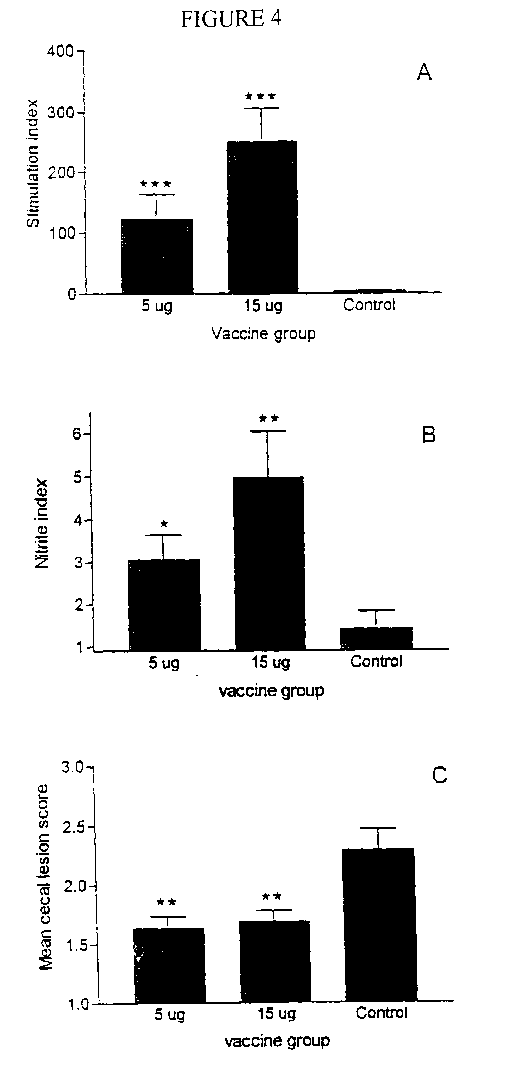

A dose-effect experiment was carried out to determine whether the level of protection could be increased. Groups of animals were vaccinated with different amounts of antigen (0, 5 and 15 µg/dose). Animals received a priming and booster vaccination with a three week interval. Eleven days after booster vaccination peripheral blood lymphocyte responsiveness against total sporozoite antigen was determined. Lymphocytes from vaccinated animals reacted upon stimulation with sporozoite antigen. There was a clear dose-effect relationship in that lymphocytes from animals that had received the highest antigen dose showed highest T-cell activation responses (in both the proliferation assay and the nitrite assay; Fig. 4A and B). When challenged with *E. tenella* sporulated oocysts four days later it appeared that there was no dose-effect relationship between the level of protection and the antigen dose used for vaccination; both groups of animals were protected to a similar degree with regard to the development of lesions in the ceca (Fig. 4C) . The reduction in the mean lesion score of both vaccinated groups of animals was statistically significant different from that of the control group (P < 0.005).

### DISCUSSION

Nine hydrophilic fractions of sporozoite proteins, separated according to different molecular weight, were tested for their ability to stimulate T-cell responses in PBL from day 8 p.i. All nine fractions induced proliferation of day 8 PBL. Although the protein concentration varied between fractions, it was decided not to correct these concentrations for the reason that each of the fractions again contained two or more different polypeptides in unknown ratios. This means that differences in the height of the responses could therefore not be relied upon entirely to select relevant fractions. Data of the NO assay were included as a second criterion to select the fractions for vaccination-challenge experiments. Apart from the fractions 1, 2 and 3 that stimulated both types of T-cell responses in PBL from infected animals, fraction 8 was included as containing relatively high proliferation inducing capacity at low MAF inducing activity (probably IFN-γ; Breed *et al.* 1997). Although, all vaccine preparations induced strong T-cell responses, surprisingly only one fraction induced partial protection against oral challenge infection with *E. tenella* oocysts.

**Figure 1** T-cell responses of PBL from *E. tenella* infected (day 8 p.i.) and control chickens against different hydrophilic fractions of *E. tenella* sporozoites. HPS or total sporozoite antigen (sporo). Fractions were tested at a concentration of 0.15 x their original volume. (A) Mean proliferative responses. (B) Mean MAF-activity by PBL supernatants. Vertical lines indicate standard errors of the mean (SEM). * Significantly different from the untreated group (* P < 0.05, ** P < 0.005, *** P < 0.0005).

**Figure 2** SDS-PAGE of the hydrophilic fractions of *E. Tenella* sporozoites used for vaccination. Stained with silver. Each set covers 3 lanes with two-fold serial dilutions of the fraction.

**Figure 3** Vaccine potential of the selected antigen fractions. PBL were taken 11 days after booster vaccination and stimulated with *E. tenella* sporozoite antigen. (A) Mean proliferative PBL responses (B). Mean MAF-activity by supernatants. (C) Mean cecal lesion score of vaccinated groups following *E. tenella* challenge infection. Vertical lines indicate SEM. * Significantly different from the untreated group (* P < 0.05, ** P < 0.005, *** P < 0.0005).

**Figure 4** Dose-effect response of antigen fraction 3. PBL were taken 11 days after booster vaccination and stimulated with *E. tenella* sporozoite antigen. (A) Mean proliferative PBL responses (B). Mean MAF-activity by supernatants. (C) Mean cecal lesion score of vaccinated groups following *E. tenella* challenge infection. Vertical lines indicate SEM. * Significantly different from the untreated group (* P < 0.05, ** P < 0.005, *** P < 0.0005).

### REFERENCES

Bennett S. & Riley E.M. (1992) The statistical analysis of data from immunoepidemiological studies. *Journal of Immunological Methods* **146**, 229-239
Beug H., Von Kirchbach A., Döderlein G., Conscience J-F. & GrafT. (1979) Chicken hematopoietic cells transformed by seven strains of defective avian leukemia viruses display three distinct phenotypes of differentiation. *Cell* **18***,* 375-390
Bordier C. (1981) Phase separation of integral membrane proteins in Triton X-114 solution. *Journal of Biological Chemistry* **256**, 1604-1607
Breed D.G.J., Dorrestein J. & Vermeulen A.N. (1996) Immunity to *Eimeria tenella* in chickens: phenotypical and functional changes in peripheral blood T-cell subsets. *Avian Diseases* **40**, 37-48
Breed D.G.J., Dorrestein J., Schetters Th.P.M., Waart van den L., Rijke E.O. & Vermeulen A.N. (1997) Pheripheral blood lymphocytes from *E. tenella* infected chickens produce gamma-interferon after stimulation *in vitro. Parasite Immunology* **19**, (127-135)
Chapman H.D. (1993) Resistance to anticoccidial drugs in fowl. *Parasitology Today* **9***,* 159-162
Danforth H.D., Augustine P.C., Ruff M.D., McCandliss R., Strausberg R.L. & Likel M. (1989) Genetically engineered antigen confers partial protection against avian coccidial parasites. *Poultry Science* **68***,* 1643-1652
Dunn P.P.J., Billington K., Bumstead J.M. & Tomley F.M. (1995) Isolation and sequences of cDNA clones for cytosolic and organellar hsp70 species in *Eimeria* spp. *Molecular and Biochemical Parasitology* **70***,* 211-215
Jenkins M.C.. Augustine P.C., Danforth H.D. & Barta J.R. (1991) X-irradiation of *Eimeria tenella* oocysts provides direct evidence that sporozoite invasion and early schizont development induce a protective immune response(s) *Infection and Immunity* **59**, 4042-4048
Jenkins M.C.. Seferian P.G., Augustine P.C. & Danforth H.D. (1993) Protective immunity against coccidiosis elicited by radiation-attenuated *Eimeria maxima* sporozoites that are incapable of asexual development. *Avian Diseases* **37**, 74-82
Johnson, J., & Reid W.M. (1970) Anticoccidial drugs: lesion scoring techniques in battery and floor-pen experiments with chickens. *Experimental Parasitology* **28**, 30-36.
Laemmli U.K. (1970) Cleavage of structural proteins during the assembly of the head of bacteriophage T4. *Nature* **227**, 680-685
Larsen R.A., Kyle J.E., Whitmire W.M. & Speer C.A. (1984) Effects of nylon wool purification on infectivity and antigenicity of *Eimeria falciformis* sporozoites and merozoites. *Journal of Parasitology* **70**, 597-601
Lillehoj H.S. & Trout J.M. (1993) Coccidia: a review of recent advances on immunity and vaccine development. *Avian Pathology* **22**, 3-31
Long P.L., Millard B.J., Joyner L.P. & Norton C.C. (1976) A guide to the laboratory techniques used in the study and diagnosis of avian coccidiosis. *Folia Veterinaria Latina* **6**, 201-217
McKenzie M.E. & Long P.L. (1986) Immunization against coccidiosis with extracts of *Eimeria*-infected tissues. *Poultry Science* **65**, 892-897
Murray P.K.. Bhogal B.S.. Crane M.S.J. & MacDonald T.T. (1986) *Eimeria tenella* - *in vivo* immunization studies with sporozoite antigen. In: *Research in Avian Coccidiosis. Proceedings of the Georgia Coccidiosis Conference* (Eds.: L.R. McDougald. Joyner L.P. and P.L. Long) Athens. University of Georgia. pp. 564-573
Rose M.E. (1996) Immunity to coccidia. In: *Poultry Immunology* (Ed.: T.F. Davison, T.R. Morris and L.N. Payne), Carfax Publishing Company, Oxfordshire, U.K. pp. 265-299
Shirley M.W. (1986) New methods for the identification of species and strains of *Eimeria.* In: *Research in Avian Coccidiosis. Proceedings of the Georgia Coccidiosis Conference* (Eds.: L.R. McDougald, Joyner L.P. and P.L. Long) Athens, University of Georgia. pp. 13-35
Shirley M.W. (1993) Live vaccines for the control of coccidiosis. In: *Proceedings of the VIth. International Coccidiosis Conference* (Eds.: J.R. Barta and M.A. Fernando) Moffitt Print Craft Ltd., Guelph. pp. 61-72
Toyama T. & Kitano N. (1983) Effect of bile salts on *in vitro* excystation of *Eimeria tenella* oocysts. *Japanese Journal of Veterinary Science* **45**, 139-141
Wray W., Boulikas T., Wray V.P. & Hannock R. (1981) Silverstaining of proteins in polyacrylamide

## Claims

1. A composition free of whole *Eimeria* parasites, which comprises one or more proteins, or fragments or variants thereof; wherein said proteins:
(a) are present in the hydrophilic phase of a Triton X-114 extract of *Eimeria* sporozoites
(b) have molecular masses of 26-30 kDa ± 5 kDa (i.e. 21-35 kDa) when determined by SDS-PAGE under reducing conditions.

2. A composition according to claim 1 wherein said extract of *Eimeria* sporozoites is an extract of *E. tenella, E. acervulina, E. maxima, E. brunetti, E. necatrix or E. mitis* sporozoites.

3. A composition according to claim 1 or claim 2; wherein at least 50% w/w of proteinaceous material present is made up of one or more of said proteins, fragments and/or variants.

4. A composition according to any preceding claim wherein a plurality (e.g. two or three of said proteins, fragments or variants thereof are present).

5. A composition according to any of claims 1 to 3 wherein only one of said proteins or fragments or variants thereof is present, e.g. in substantially pure form.

6. A nucleic acid molecule, which:
a) encodes a protein, variant or fragment thereof, as described in any of claims 1 to 5,
b) is complementary to a nucleic acid molecule as described in a),
or
c) hybridises to a nucleic acid molecule as described in a) or b).

7. A nucleic acid molecule according to claim 6, which is in isolated or recombinant form.

8. A vector comprising a nucleic acid molecule according to claim 6 or claim 7.

9. A non-avian host comprising a vector according to claim 8 or a nucleic acid according to claim 6 or claim 7.

10. A vector according to claim 8 or a host according to claim 9, which is adapted to express a protein, variant or fragment thereof as described in any of claims 1 to 5.

11. A pharmaceutically acceptable vaccine composition comprising a vector or host according to claim 10 (whether in live, killed or attenuated form).

12. A pharmaceutically acceptable composition according to any of claims 1 to 5 in the form of a vaccine.

13. A composition according to claim 11 or claim 12 wherein said vaccine comprises an adjuvant.

14. A composition according to claim 12 wherein the adjuvant is Quil A.

15. A composition according to any of claims 12 to 14, which is in unit dosage form.

16. A composition according to any of claims 1 to 5 or claims 11 to 15 for use in medicine.

17. The use of a composition according to any of claims 1 to 5 in the preparation of a vaccine against an *Eimeria*-mediated disorder, e.g. against coccidiosis.

18. An antibody or a derivative thereof that binds with a protein, variant or fragment thereof as described in any of claims 1 to 5.

19. An immunological reagent comprising a protein, variant or fragment thereof as described in any of claims 1 to 5 bound to a support or provided with a detectable label.

20. An immunological reagent comprising a protein, variant or fragment thereof as disclosed in any of claims 1 to 5, which is bound to a support or provided with a labelling substance.

21. A test kit for the diagnosis of *Eimeria* infection comprising a nucleic acid molecule according to claim 6 or claim 7; an antibody or derivative thereof according to claim 18; or an immunological reagent according to claim 19 or claim 20.
